# EUROPÄISCHE PATENTSCHRIFT

(11) **EP 0 703 224 B1**
(45) Veröffentlichungstag und Bekanntmachung des Hinweises auf die Patenterteilung: **03.09.1997**
(21) Anmeldenummer: 95114213.2
(22) Anmeldetag: 11.09.1995
(51) Int. Cl.: C07D 249/12

(54) **Verfahren zur Herstellung von Alkoxytriazolinonen**
Process for the preparation of alkoxytriazolinones
Procédé de préparation d'alcoxytriazolinones

(30) Priorität: 23.09.1994 DE 4433968
(43) Veröffentlichungstag der Anmeldung: 27.03.1996
(73) Patentinhaber: BAYER AG, 51368 Leverkusen (DE)
(72) Erfinder: Wroblowsky, Heinz-Jürgen, Dr., D-40764 Langenfeld (DE); König, Klaus, Dr., D-51519 Odenthal (DE); Kluth, Joachim, Dr., D-40764 Langenfeld (DE); Müller, Klaus-Helmut, Dr., D-40593 Düsseldorf (DE)

(56) Entgegenhaltungen:
- EP-A- 0 477 646
- EP-A- 0 507 171
- JOURNAL OF THE CHEMICAL SOCIETY, PERKIN TRANSACTIONS I, 1973 LONDON GB, Seiten 2644-6, P.R. ATKINS ET AL. 'Heterocyclic syntheses with isothiocyanatoformic esters and their derivatives'
- ARCHIV DER PHARMAZIE, Bd. 320, Nr. 7, Juli 1987 WEINHEIM DE, Seiten 608-16, J. BONJEAN ET AL. 'Isolamtidin und Analoge'

## Beschreibung

Die Erfindung betrifft ein neues, auch technisch anwendbares Verfahren zur Herstellung von weitgehend bekannten Alkoxytriazolinonen, welche als Zwischenprodukte zur Herstellung von agrochemischen Wirkstoffen verwendet werden können.

Alkoxytriazolinone und mehrere Methoden zu ihrer Herstellung sind bereits bekannt (vgl. J. Indian Chem. Soc. 6 (1929), 565-575; J. Chem. Soc. Perkin I 1973, 2644-2646; Arch. Pharm. 307 (1974), 889-891; EP-A 477646; EP-A 507171). Nach diesen bekannten Synthesemethoden werden Alkoxytriazolinone jedoch nur in sehr unbefriedigenden Ausbeuten erhalten.

Ferner ist bekannt, daß durch Methylierung von Urazol oder 4-Methylurazol mit Diazomethan (CH₂N₂) 5-Methoxy-4-methyl-2,4-dihydro-3H-1,2,4-triazol-3-on gebildet wird [vgl. F. Arndt et al, Rev. Fac. Sci. Istanbul 13A, S. 127-144 (1948)]; diese Methode liefert zwar das Triazolinon in hoher Ausbeute, kann aber technisch nicht durchgeführt werden.

Es wurde nun gefunden, daß man Alkoxytriazolinone der allgemeinen Formel (I), 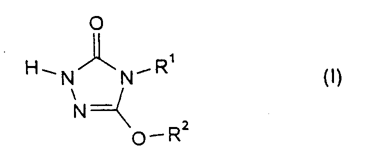 in welcher
- R¹: für jeweils gegebenenfalls substituiertes Alkyl, Alkenyl, Alkinyl, Cycloalkyl, Cycloalkylalkyl, Aryl oder Arylalkyl steht und
- R²: für jeweils gegebenenfalls substituiertes Alkyl, Alkenyl, Alkinyl, Cycloalkyl, Cycloalkylalkyl, Aryl oder Arylalkyl steht,
in sehr guten Ausbeuten und in hoher Reinheit erhält, wenn man Iminothiokohlensäurediester der allgemeinen Formel (II) 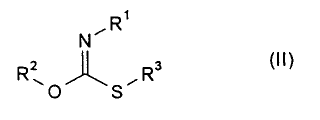 in welcher
- R¹ und R²: die oben angegebenen Bedeutungen haben und
- R³: für jeweils gegebenenfalls substituiertes Alkyl, Aryl oder Arylalkyl steht,
mit Carbazinsäureestern der allgemeinen Formel (III) 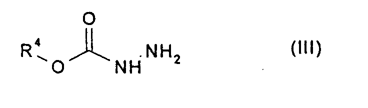 in welcher
- R⁴: für jeweils gegebenenfalls substituiertes Alkyl, Aryl oder Arylalkyl steht,
- oder mit Säureaddukten von Verbindungen der Formel (III) -
gegebenenfalls in Gegenwart eines Reaktionshilfsmittels und gegebenenfalls in Gegenwart eines Verdünnungsmittels bei Temperaturen zwischen -20°C und +50°C umsetzt ("erste Umsetzungsstufe") und die hierbei gebildeten Semicarbazidderivate der allgemeinen Formel (IV) 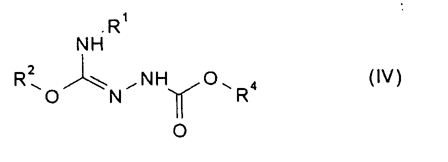 in welcher
- R¹, R² und R⁴: die oben angegebenen Bedeutungen haben,
- und/oder die entsprechenden hierzu tautomeren Verbindungen -,
gegebenenfalls nach Zwischenisolierung, gegebenenfalls in Gegenwart einer Base und gegebenenfalls in Gegenwart eines Verdünnungsmittels bei Temperaturen zwischen 20°C und 100°C cyclisierend kondensiert ("zweite Umsetzungsstufe").

Überraschenderweise können die Alkoxytriazolinone der allgemeinen Formel (I) nach dem erfindungsgemäßen Verfahren in erheblich höheren Ausbeuten als nach den meisten bekannten Synthesemethoden erhalten werden.

Gegenüber der "Diazomethan-Methode" (F. Arndt et al, l.c.) hat das erfindungsgemäße Verfahren den entscheidenden Vorteil, daß es auch technisch durchführbar ist.

Es ist hierbei vor allem als überraschend anzusehen, daß in der ersten Umsetzungsstufe praktisch ausschließlich die Abspaltung eines Mercaptans und nicht eines Alkohols erfolgt und damit eine saubere Steuerung der Reaktion möglich ist. Im Gegensatz zum bekannten Verfahren, welches bei höheren Temperaturen durchzuführen ist und bei welchem in der zweiten Umsetzungsstufe Phenol als Koppelprodukt entsteht - R⁴ steht für Phenyl (vgl. EP-A 507171, Beispiele II-1 und II-2) - kann das erfindungsgemäße Verfahren in der zweiten Stufe auch problemlos unter Abspaltung von einfachen Alkanolen durchgeführt werden, die wesentlich einfacher und mit geringerem Aufwand als Phenol zurückgewonnen werden können.

Das erfindungsgemäße Verfahren stellt somit eine wertvolle Bereicherung des Standes der Technik dar.

Die Erfindung betrifft vorzugsweise die Herstellung von Verbindungen der Formel (I), in welcher
- R¹: für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht und
- R²: für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht.

Die Erfindung betrifft insbesondere die Herstellung von Verbindungen der Formel (I), in welcher
- R¹: für jeweils gegebenenfalls durch Fluor, Chlor und/oder Brom, Methoxy oder Ethoxy substituiertes Methyl, Ethyl, n- oder i-Propyl, n-, i-, s-oder t-Butyl, für jeweils gegebenenfalls durch Fluor, Chlor und/oder Brom substituiertes Propenyl, Butenyl, Propinyl oder Butinyl, für jeweils gegebenenfalls durch Fluor, Chlor, Methyl oder Ethyl substituiertes Cyclopropyl, Cyclobutyl oder Cyclopropylmethyl, oder für jeweils gegebenenfalls durch Fluor, Chlor, Brom, Methyl, Ethyl, Trifluormethyl, Methoxy, Ethoxy, Difluormethoxy, Trifluormethoxy, Methoxycarbonyl oder Ethoxycarbonyl substituiertes Phenyl oder Benzyl steht und
- R²: für jeweils gegebenenfalls durch Fluor, Chlor und/oder Brom, Methoxy oder Ethoxy substituiertes Methyl, Ethyl, n- oder i-Propyl, n-, i-, s-oder t-Butyl, für jeweils gegebenenfalls durch Fluor, Chlor und/oder Brom substituiertes Propenyl, Butenyl, Propinyl oder Butinyl, für jeweils gegebenenfalls durch Fluor, Chlor, Methyl oder Ethyl substituiertes Cyclopropyl, Cyclobutyl oder Cyclopropylmethyl, oder für jeweils gegebenenfalls durch Fluor, Chlor, Brom, Methyl, Ethyl, Trifluormethyl, Methoxy, Ethoxy, Difluormethoxy, Trifluormethoxy, Methoxycarbonyl oder Ethoxycarbonyl substituiertes Phenyl oder Benzyl steht.

Verwendet man beispielsweise Methylimino-thiokohlensäure-O,S-dimethylester und Carbazinsäure-ethylester als Ausgangsstoffe, so kann der Reaktionsablauf beim erfindungsgemäßen Verfahren durch das folgende Formelschema skizziert werden:

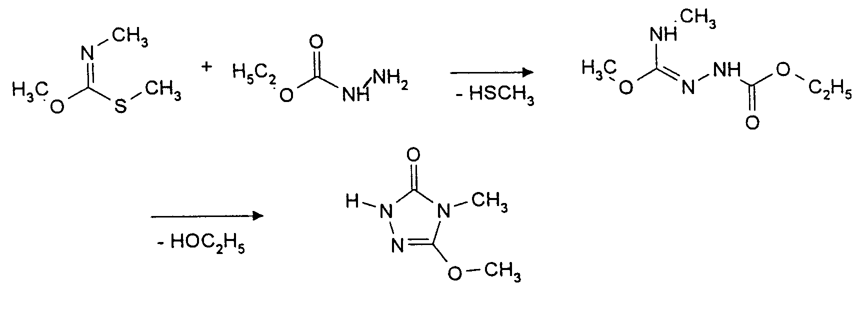

Die beim erfindungsgemäßen Verfahren zur Herstellung der Verbindungen der allgemeinen Formel (I) als Ausgangsstoffe zu verwendenden Iminothiokohlensäurediester sind durch die Formel (II) allgemein definiert. In der Formel (II) haben R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹ und R² angegeben wurden; R³ steht vorzugsweise für gegebenenfalls durch Carboxy oder C₁-C₄-Alkoxy-carbonyl substituiertes C₁-C₄-Alkyl, für Phenyl oder Benzyl, insbesondere für Methyl, Ethyl, Carboxymethyl, Methoxycarbonylmethyl oder Ethoxycarbonylmethyl.

Die Ausgangsstoffe der Formel (II) sind bekannt und/oder können nach an sich bekannten Verfahren hergestellt werden (vgl. Chem. Ber. 120 (1987), 339-344).

Die beim erfindungsgemäßen Verfahren weiter als Ausgangsstoffe zu verwendenden Carbazinsäureester sind durch die Formel (III) allgemein definiert. In der Formel (III) steht R⁴ vorzugsweise für gegebenenfalls durch C₁-C₄-Alkoxy substituiertes C₁-C₄-Alkyl, für Phenyl oder Benzyl, insbesondere für Methyl, Ethyl, Methoxyethyl, Ethoxyethyl oder Phenyl.

Die Ausgangsstoffe der Formel (III) sind bekannte organische Synthesechemikalien.

Es können vorteilhaft auch Säureaddukte von Verbindungen der Formel (III) zur Durchführung des erfindungsgemäßen Verfahrens eingesetzt werden. Solche sind vorzugsweise Addukte mit starken Protonensäuren, wie z.B. Hydrogenchlorid ("Salzsäure"), Hydrogenbromid, Hydrogeniodid, Schwefelsäure, Methansulfonsäure, Benzolsulfonsäure und p-Toluolsulfonsäure.

Als Verdünnungsmittel zur Durchführung des erfindungsgemäßen Verfahrens kommen (in den beiden Umsetzungsstufen) die üblichen organischen Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n-oder i-Propanol, n-, i-, s- oder t-Butanol, Ethylenglykolmonomethylether, Ethylenglykolmonoethylether, Diethylenglykolmonomethylether, Diethylenglykolmonoethylether, sowie deren Gemische mit Wasser oder reines Wasser.

Alkohole, wie Methanol, Ethanol, n- oder i-Propanol, deren Gemische mit Wasser sowie reines Wasser werden als Verdünnungsmittel besonders bevorzugt.

Das erfindungsgemäße Verfahren wird in der ersten Stufe vorzugsweise in Gegenwart eines geeigneten Reaktionshilfsmittels durchgeführt. Als solche kommen vorzugsweise Protonensäuren, wie z.B. Salzsäure, Schwefelsäure, Phosphorsäure, Kohlensäure, Essigsäure, Propionsäure, Pivalinsäure, Methansulfonsäure, Benzoesäure, Benzolsulfonsäure und p-Toluolsulfonsäure - gegebenenfalls auch polymere Säuren oder saure Ionenaustauscher - in Betracht.

Pivalinsäure, Essigsäure und (wässrige) Salzsäure werden als Reaktionshilfsmittel bei der ersten Stufen des erfindungsgemäßen Verfahrens besonders bevorzugt.

Vorzugsweise können auch unmittelbar Säureaddukte von Verbindungen der Formel (III) eingesetzt werden.

Das erfindungsgemäße Verfahren wird in der zweiten Stufe gegebenenfalls in Gegenwart einer Base durchgeführt. Es kommen hierbei alle üblichen anorganischen oder organischen Basen in Betracht. Hierzu gehören beispielsweise Erdalkali-oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie basische organische Stickstoffverbindungen, wie Trimethylamin, Triethylamin, Tributylamin, N,N-Dimethylanilin, N,N-Dimethyl-benzylamin, Pyridin, N-Methylpiperidin, N,N-Dimethylaminopyridin, 5-Ethyl-2-methyl-pyridin, Diazabicyclooctan (DABCO), Diazabicyclononen (DBN) oder Diazabicycloundecen (DBU).

Alkalimetallalkoholate, wie Natriummethylat oder Natriumethylat, sowie Alkalimetallhydroxide, wie Natrium- oder Kaliumhydroxid, vorzugsweise in den entsprechenden Alkoholen bzw. in Wasser gelöst, werden als Basen bei der zweiten Stufe des erfindungsgemäßen Verfahrens besonders bevorzugt.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen -20°C und +50°C, vorzugsweise bei Temperaturen zwischen -10°C und +40°C, insbesondere bei Temperaturen zwischen 0°C und 30°C.

Die Reaktionstemperaturen können bei der Durchführung der zweiten Stufe des erfindungsgemäßen Verfahrens in einem größeren Bereich variiert werden. Im allgemeinen arbeitet man bei Temperaturen zwischen 20°C und 100°C, vorzugsweise bei Temperaturen zwischen 30°C und 90°C, insbesondere bei Temperaturen zwischen 40°C und 80°C.

Das erfindungsgemäße Verfahren wird in beiden Stufen im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens zur Herstellung der Verbindungen der Formel (I) setzt man pro Mol Iminothiokohlensäurediester der Formel (II) im allgemeinen 0,5 bis 1,2 Mol, vorzugsweise 0,8 bis 1,1 Mol Carbazinsäureester der Formel (III) und gegebenenfalls 0,001 bis 2,0 Mol, vorzugsweise 0,01 bis 1,0 Mol Reaktionshilfsmittel ein.

In einer bevorzugten Ausführungsform des erfindungsgemäßen Verfahrens werden die Ausgangsstoffe der Formel (II) und der Formel (III) - von letzteren gegebenenfalls deren Säureaddukte - sowie gegebenenfalls ein Reaktionshilfsmittel in einem geeigneten Verdünnungsmittel vermischt und bei der erforderlichen Temperatur gerührt, bis praktisch kein Ausgangsmaterial mehr vorhanden ist. Die Isolierung des Zwischenproduktes der Formel (IV) kann dann auf übliche Weise, beispielsweise durch Einengen, Digerieren des Rückstandes mit einem organischen Lösungsmittel, wie z.B. Diethylether, Methyl-t-butylether oder Essigsäureethylester, und Absaugen durchgeführt werden. Das Zwischenprodukt der Formel (IV) kann aber auch ohne Zwischenisolierung mit einer Base - gegebenenfalls in einem der oben angegebenen Verdünnungsmittel gelöst - versetzt und bei der zur cyclisierenden Kondensation erforderlichen Temperatur bis zum Ende der Umsetzung gerührt werden.

Die Aufarbeitung zur Isolierung der Produkte der Formel (I) kann nach üblichen Methoden durchgeführt werden. Beispielsweise wird eingeengt, in Wasser aufgenommen und - z.B. mit Salzsäure - neutralisiert oder angesäuert. Sofern das Produkt hierbei kristallin anfällt, wird es durch Absaugen isoliert. Andernfalls wird mit einem mit Wasser praktisch nicht mischbaren organischen Lösungsmittel, wie z.B. Essigsäureethylester, geschüttelt, die organische Phase - z.B. mit Magnesiumsulfat - getrocknet und filtriert. Nach sorgfältigem Abdestillieren des Lösungsmittels unter vermindertem Druck wird dann das Produkt der Formel (I) als Rückstand erhalten. Die Rohprodukte können durch Umkristallisation, Verrühren mit einem geeigneten organischen Lösungsmittel, wie z.B. Petrolether, oder durch Destillation gereinigt werden.

Die nach dem erfindungsgemäßen Verfahren herzustellenden Verbindungen der Formel (I) können als Zwischenprodukte zur Herstellung von herbizid wirksamen Verbindungen verwendet werden (vgl. EP-A 477646 und EP-A 507171).

### Herstellungsbeispiele:

### Beispiel 1

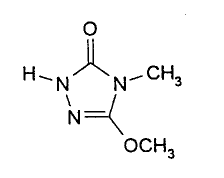

12 g (85 mMol) Carbazinsäure-ethylester-hydrochlorid werden bei 0°C bis 5°C in Methanol vorgelegt und bei dieser Temperatur wird eine Lösung von 10,5 g (88 mMol) Methyliminothiokohlensäure-O,S-dimethylester in 15 ml Methanol zugetropft. Die Mischung wird 3 Stunden bei 0°C bis 5°C gerührt und dabei wird das als Koppelprodukt gebildete Methylmercaptan mit Stickstoff ausgeblasen. Anschließend werden bei 0°C bis 5°C 15,5 g einer Lösung von Natriummethanolat in Methanol (86 mMol NaOCH₃) zugetropft und die Reaktionsmischung wird 30 Minuten bei ca. 5°C, weitere 30 Minuten bei ca. 20°C und - nach Zugabe von weiteren 15,5 g der oben angegebenen Natriummethanolat-Lösung - weitere 6 Stunden bei 50°C gerührt. Dann wird im Wasserstrahlvakuum eingeengt, der Rückstand in 60 ml gesättigter Kochsalzlösung aufgenommen, unter Eiskühlung mit 20%iger Salzsäure angesäuert und das kristallin angefallenen Produkt durch Absaugen isoliert. Das so erhaltene salzhaltige Produkt wird in 25 ml heißem Wasser gelöst, dann 4 Stunden bei 20°C gerührt und erneut abgesaugt.

Man erhält 9,9 g 5-Methoxy-4-methyl-2,4-dihydro-3H-1,2,4-triazol-3-on (83%ig, Rest: Natriumchlorid); Ausbeute: 74,5% der Theorie.

### Beispiel 2

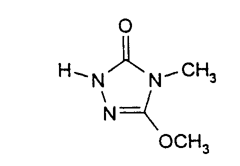

21,4 g (0,20 Mol) Carbazinsäure-ethylester und 2,2 g (0,02 Mol) Carbazinsäureethylester-hydrochlorid werden in 80 ml Methanol vorgelegt und 29,3 g (0,24 Mol) Methyliminothiokohlensäure-O,S-dimethylester werden dazu gegeben. Die Mischung wird dann 4 Stunden bei 20°C gerührt. Anschließend werden 43 g einer Lösung von Natriummethanolat in Methanol (0,24 Mol NaOCH₃) dazu gegeben und die Reaktionsmischung wird 7 Stunden bei 60°C gerührt. Zur Aufarbeitung wird eingeengt, der Rückstand in 95 ml Wasser aufgenommen und unter Eiskühlung mit konz. Salzsäure neutralisiert. Das kristallin angefallenen Produkt wird durch Absaugen isoliert.

Man erhält 21,7 g (70% der Theorie) 5-Methoxy-4-methyl-2,4-dihydro-3H-1,2,4-triazol-3-on vom Schmelzpunkt 148°C.

### Beispiel 3

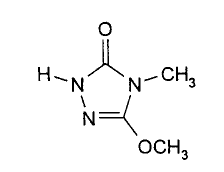

21,4 g (0,20 Mol) Carbazinsäure-ethylester werden in 70 ml Wasser vorgelegt und bei ca. 5°C mit 4 g konz. Salzsäure (0,04 Mol HCl) versetzt. Dann werden 26,6 g (0,22 Mol) Methyliminothiokohlensäure-O,S-dimethylester dazu gegeben. Die Mischung wird dann 4 Stunden bei 5°C gerührt. Anschließend werden 21,3 g einer 45%igen Lösung von Natriumhydroxid in Wasser (0,24 Mol NaOH) dazu gegeben und die Reaktionsmischung wird 4 Stunden bei 55°C bis 60°C gerührt. Zur Aufarbeitung wird unter Eiskühlung mit konz. Salzsäure neutralisiert. Das kristallin angefallene Produkt wird durch Absaugen isoliert.

Man erhält 23,2 g (89% der Theorie) 5-Methoxy-4-methyl-2,4-dihydro-3H-1,2,4-triazol-3-on vom Schmelzpunkt 148°C.

### Beispiel 4

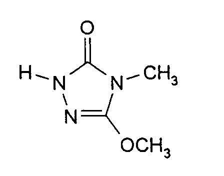

6,7 g (0,03 Mol) N'-(α-Methylamino-α-methoxy-methylen)-hydrazin-N-carbonsäure-phenylester werden bei 0°C in eine Mischung aus Ethanol und 48 ml 3N-Natronlauge eingetragen und das Reaktionsgemisch wird noch eine Stunde bei 0°C gerührt. Dann wird mit Eis versetzt, mit konz. Salzsäure angesäuert und mit Kochsalz gesättigt. Das Reaktionsprodukt wird mit Essigsäureethylester extrahiert, die organische Phase mit Natriumsulfat getrocknet und filtriert. Das Filtrat wird eingeengt, der Rückstand mit Diisopropylether verrührt und das kristallin angefallene Produkt durch Absaugen isoliert.

Man erhält 3,1 g (80% der Theorie) 5-Methoxy-4-methyl-2,4-dihydro-3H-1,2,4-triazol-3-on vom Schmelzpunkt 148°C.

### Beispiel 5

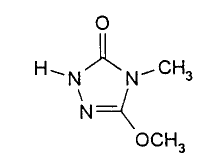

21,2 g (0,2 Mol) Carbazinsäure-ethylester werden in 40 ml Wasser vorgelegt und bei 0°C werden 4,0 g konz. Salzsäure (0,04 Mol HCl) zugetropft. Dann wird bei 0°C eine Lösung von 26,2 g (0,22 Mol) Methyliminothiokohlensäure-O,S-dimethylester in 30 ml Methanol tropfenweise dazu gegeben. Das Reaktionsgemisch wird 17 Stunden im Eisbad gerührt und das als Koppelprodukt gebildete Methylmercaptan wird dabei mit Stickstoff ausgetrieben. Anschließend werden 21,3 g 45%ige wässrige Natronlauge (0,24 Mol NaOH) zugetropft und die Mischung wird dann 3 Stunden auf 55°C erwärmt. Nach dem Abkühlen wird das Methanol im Wasserstrahlvakuum abdestilliert, der Rückstand mit 90 ml Wasser verdünnt und im Eisbad durch Zugabe von konz. Salzsäure neutralisiert. Das hierbei kristallin angefallene Produkt wird durch Absaugen isoliert.

Man erhält 24 g 5-Methoxy-4-methyl-2,4-dihydro-3H-1,2,4-triazol-3-on (95,2%ig, Ausbeute: 89% der Theorie).

### Beispiel 6

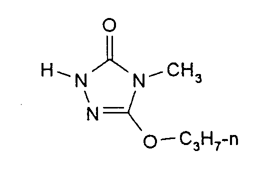

21,1 g (0,2 Mol) Carbazinsäure-ethylester werden in 70 ml Wasser vorgelegt und nach Zugabe von 4 g konz. Salzsäure (0,04 Mol HCl) auf 0°C abgekühlt. Dann werden 32,9 g (0,22 Mol) Methyliminothiokohlensäure-O-n-propylester-S-methylester zugetropft und die Mischung wird noch 15 Stunden unter Eiskühlung gerührt, wobei das als Koppelprodukt entstandene Methylmercaptan mit Stickstoff ausgetrieben wird. Anschließend werden bei 20°C 21,3 g 45%ige Natronlauge (0,22 Mol NaOH) dazu gegeben und die Mischung wird 6 Stunden auf 60°C erwärmt.

Dann wird unter Eiskühlung durch Zugabe von konz. Salzsäure neutralisiert und dreimal mit je 100 ml Essigsäureethylester extrahiert. Die vereinigten Extraktionslösungen werden mit Natriumsulfat getrocknet und filtriert. Das Filtrat wird im Wasserstrahlvakuum eingeengt, der Rückstand mit Petrolether verrührt und das kristallin angefallene Produkt durch Absaugen isoliert.

Man erhält 26,7 g (84% der Theorie) 5-n-Propoxy-4-methyl-2,4-dihydro-3H-1,2,4-triazol-3-on vom Schmelzpunkt 77°C.

### Beispiel 7

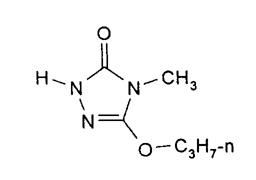

21,2 g (0,2 Mol) Carbazinsäure-ethylester werden in 40 ml Wasser vorgelegt und bei 0°C werden 4 g konz. Salzsäure (0,04 Mol HCl) eingetropft. Dann wird bei 0°C innerhalb von 50 Minuten eine Lösung von 33,8 g (0,22 Mol) Methyliminothiokohlensaure-O-n-propylester-S-methylester in 30 ml Methanol tropfenweise dazu gegeben und die Mischung wird noch 6 Stunden bei 0°C und weitere 15 Stunden bei 20°C gerührt, wobei das als Koppelprodukt gebildete Methylmercaptan mit Stickstoff ausgetrieben wird. Dann werden 21,3 g 45%ige wässrige Natronlauge (0,24 Mol NaOH) eingetropft und die Mischung wird 6 Stunden bei 55°C bis 60°C gerührt. Anschließend wird im Wasserstrahlvakuum eingeengt und der Rückstand mit konz. Salzsäure neutralisiert. Man extrahiert dreimal mit je 100 ml Essigsäureethylester, trocknet die vereinigten Extraktionslösungen mit Natriumsulfat und filtriert. Das Filtrat wird eingeengt, der Rückstand mit Petrolether verrieben und das kristallin erhaltene Produkt durch Absaugen isoliert.

Man erhält 29,7 g (92% der Theorie) 5-n-Propoxy-4-methyl-2,4-dihydro-3H-1,2,4-triazol-3-on vom Schmelzpunkt 77°C.

### Beispiel 8

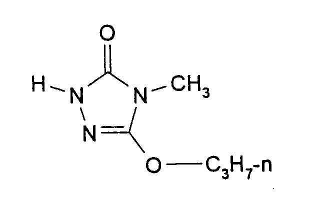

Eine Mischung aus 95,5 g (0,5 Mol) Methyliminothiokohlensäure-O-n-propylester-S-(2-methoxylethyl)-ester, 76 g (0,5 Mol) Carbazinsäure-phenylester und 47 g (0,5 Mol) Phenol wird 10 Stunden bei 20°C und weitere 3 Stunden bei 60°C gerührt. Danach wird eingeengt und bei 1 mbar destilliert.

Man erhält 47,5 g eines Rohdestillats, das zu 79,2 % aus dem gewünschten 5-n-Propoxy-4-methyl-2,4-dihydro-3H-1,2,4-triazol-3-on besteht (Ausbeute: 47,9 % der Theorie).

### Beispiel 9

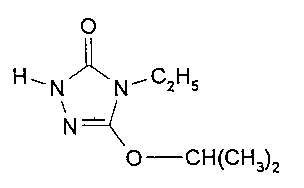

Analog zu Beispiel 3 erhält man durch Umsetzung von etwa äquimolaren Mengen von Carbazinsäure-ethylester und Ethylimino-thiokohlensäure-O-isopropylester-S methylester (10 Mol-% Überschuß) in Gegenwart von Pivalinsäure (50 Mol-%, anstelle von 20 Mol-% HCl) und anschließende Umsetzung des gebildeten Zwischenproduktes mit NaOCH₃ (anstelle von NaOH) 4-Ethyl-5-isopropoxy-2,4-dihydro-3H-1,2,4-triazol-3-on (Ausbeute: 62 % d. Th.) vom Schmelzpunkt 69 bis 70°C (nach Destillation).

### Beispiel 10

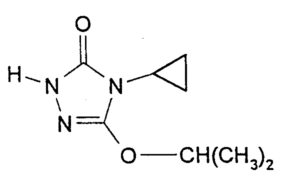

Analog zu den Beispielen 3 und 9, wobei jedoch 100 Mol-% Pivalinsäure eingesetzt werden, erhält man 4-Cyclopropyl-5-isopropoxy-2,4-dihydro-3H-1,2,4-triazol-3-on (Ausbeute: 64 % d. Th.) vom Schmelzpunkt 146 bis 147°C (umkristallisiert aus Wasser).

### Zwischenprodukte der Formel (IV):

### Beispiel (IV-1)

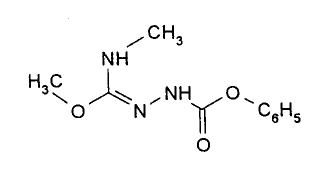

5,66 g (0,03 Mol) Carbazinsäure-phenylester-hydrochlorid werden in 50 ml Methanol vorgelegt und nach Abkühlen auf 0°C bis 5°C werden 4,24 g (0,035 Mol) Methyliminothiokohlensäure-O,S-dimethylester dazu gegeben. Das Gemisch wird 2 Stunden bei 0°C bis 5°C gerührt und das als Koppelprodukt gebildete Methylmercaptan wird mit Stickstoff ausgetrieben. Dann werden bei 0°C 3.04 g (0,03 Mol) Triethylamin zugetropft und anschließend wird im Wasserstrahlvakuum eingeengt. Der Rückstand wird mit wenig Wasser verrührt und das kristalline Produkt durch Absaugen isoliert.

Man erhält 5,5 g (82% der Theorie) N'-(α-Methylamino-α-methoxy-methylen)-hydrazin-N-carbonsäure-phenylester vom Schmelzpunkt 129°C (unter Zersetzung).

### Beispiel (IV-2)

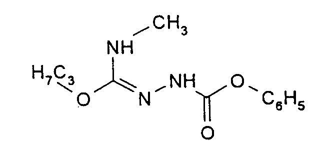

20,0 g (0,106 Mol) Carbazinsäure-phenylester-hydrochlorid werden in 200 ml Methanol vorgelegt und nach Abkühlen auf 0°C bis 5°C werden 16.3 g (0,106 Mol) Methyliminothiokohlensäure-O-propylester-S-methylester dazu gegeben. Das Gemisch wird 4 Stunden bei 0°C bis 5°C gerührt und das als Koppelprodukt gebildete Methylmercaptan mit Stickstoff ausgetrieben. Dann werden bei 0°C bis 5°C 10,8 g (0,106 Mol) Triethylamin zugetropft und anschließend wird im Wasserstrahlvakuum eingeengt. Der Rückstand wird mit 300 ml Essigsäureethylester verrührt, abgesaugt, die Mutterlauge eingeengt, der verbleibende Rückstand mit Petrolether verrührt und das kristallin angefallene Produkt durch Absaugen isoliert.

Man erhält 24,5 g (92% der Theorie) N'-(α-Methylamino-α-propoxy-methylen)-hydrazin-N-carbonsäure-phenylester.

### Beispiel (IV-3)

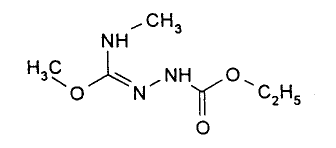

12,0 g (85 mMol) Carbazinsäure-ethylester-hydrochlorid werden in 120 ml Methanol vorgelegt und bei 0°C bis 5°C werden 10,5 g (88 mMol) Methyliminothiokohlensäure-O,S-dimethylester dazu gegeben. Das Gemisch wird 2 Stunden bei 0°C gerührt und das als Koppelprodukt gebildete Methylmercaptan wird mit Stickstoff ausgetrieben.. Dann werden bei 5°C 7,22 g (86 mMol) Natriumhydrogencarbonat dazu gegeben und nach zweistündigem Rühren wird abgesaugt. Das Filtrat wird im Wasserstrahlvakuum eingeengt, der Rückstand mit 200 ml Essigsäureethylester verrührt und das kristallin angefallene Produkt durch Absaugen isoliert.

Man erhält 11,95 g (80% der Theorie) N'-(α-Methylamino-α-methoxy-methylen)-hydrazin-N-carbonsäure-ethylester vom Schmelzpunkt 128°C.

### Beispiel (IV-4)

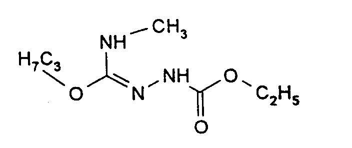

35,0 g (0,25 Mol) Carbazinsäure-ethylester-hydrochlorid werden in 500 ml Methanol vorgelegt und nach Abkühlen auf 0°C werden 38,7 g (0,25 Mol) Methyliminothiokohlensäure-O-propylester-S-methylester dazu gegeben. Das Gemisch wird 2,5 Stunden bei 5°C gerührt und das als Koppelprodukt gebildete Methylmercaptan wird mit Stickstoff ausgetrieben. Dann werden bei 0°C 34,75 ml (0,25 Mol) Triethylamin zugetropft und nach 20 Minuten wird im Wasserstrahlvakuum eingeengt. Der Rückstand wird mit 300 ml Aceton verrührt, abgesaugt, das Filtrat eingeengt, der Rückstand mit 300 ml Petrolether verrührt und das kristallin angefallenen Produkt durch Absaugen isoliert.

Man erhält 45,9 g (91% der Theorie) N'-(α-Methylamino-α-propoxy-methylen)-hydrazin-N-carbonsäure-ethylester vom Schmelzpunkt 104°C.

### Beispiel (IV-5)

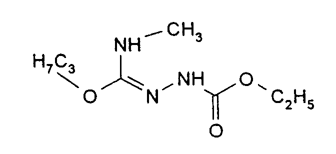

58,9 g (0,192 Mol) Carbasinsäure-ethylester-dihydrogensulfat werden in 350 ml Methanol vorgelegt und nach Abkühlen auf 0°C bis 5°C werden 60,35 g (0,39 Mol) Methyliminothiokohlensäure-O-propylester-S-methylester dazu gegeben. Das Gemisch wird 3 Stunden bei 0°C bis 5°C gerührt und das als Koppelprodukt gebildete Methylmercaptan wird mit Stickstoff ausgetrieben. Dann werden bei 0°C bis 5°C 32,76 g (0,39 Mol) Natriumhydrogencarbonat dazu gegeben und nach 30 Minuten bei 0°C bis 5°C und weiteren 2 Stunden bei 20°C wird über Kieselgur abgesaugt und vom Filtrat das Lösungsmittel unter vermindertem Druck sorgfältig abdestilliert.

Man erhält 73,5 g (94% der Theorie) N'-(α-Methylamino-α-propoxy-methylen)-hydrazin-N-carbonsäure-ethylester vom Schmelzpunkt 104°C.

## Patentansprüche

1. Verfahren zur Herstellung von Alkoxytriazolinonen der allgemeinen Formel (I), 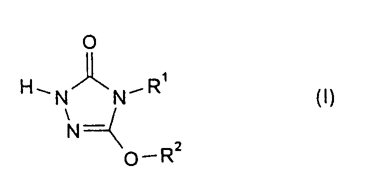 in welcher
R¹ für jeweils gegebenenfalls substituiertes Alkyl, Alkenyl, Alkinyl, Cycloalkyl, Cycloalkylalkyl, Aryl oder Arylalkyl steht und
R² für jeweils gegebenenfalls substituiertes Alkyl, Alkenyl, Alkinyl, Cycloalkyl, Cycloalkylalkyl, Aryl oder Arylalkyl steht,
dadurch gekennzeichnet, daß man Iminothiokohlensäurediester der allgemeinen Formel (II) 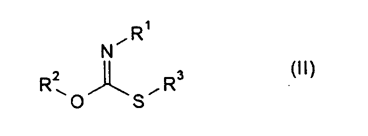 in welcher
R¹ und R² die oben angegebenen Bedeutungen haben und
R³ für jeweils gegebenenfalls substituiertes Alkyl, Aryl oder Arylalkyl steht,
mit Carbazinsäureestern der allgemeinen Formel (III) 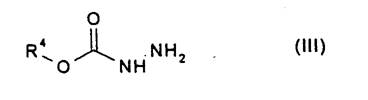 in welcher
R⁴ für jeweils gegebenenfalls substituiertes Alkyl, Aryl oder Arylalkyl steht,
- oder mit Säureaddukten von Verbindungen der Formel (III) -
gegebenenfalls in Gegenwart eines Reaktionshilfsmittels und gegebenenfalls in Gegenwart eines Verdünnungsmittels bei Temperaturen zwischen -20°C und +50°C umsetzt ("erste Umsetzungsstufe") und die hierbei gebildeten Semicarbazid-derivate der allgemeinen Formel (IV) 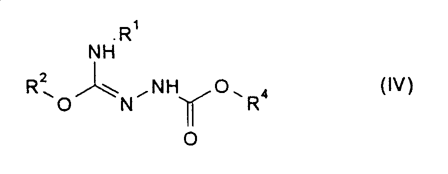 in welcher
R¹, R² und R⁴ die oben angegebenen Bedeutungen haben,
- und/oder die entsprechenden hierzu tautomeren Verbindungen -,
gegebenenfalls nach Zwischenisolierung, gegebenenfalls in Gegenwart einer Base und gegebenenfalls in Gegenwart eines Verdünnungsmittels bei Temperaturen zwischen 20°C und 100°C cyclisierend kondensiert ("zweite Umsetzungsstufe").

2. Verfahren nach Anspruch 1, dadurch gekennzeichnet, daß Verbindungen der Formel (I) hergestellt werden, in welcher
R¹ für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht und
R² für jeweils gegebenenfalls durch Halogen oder C₁-C₄-Alkoxy substituiertes Alkyl, Alkenyl oder Alkinyl mit jeweils bis zu 6 Kohlenstoffatomen, für jeweils gegebenenfalls durch Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 4 Kohlenstoffatomen im Alkylteil, oder für jeweils gegebenenfalls durch Carboxy, Cyano, Nitro, Halogen, C₁-C₄-Alkyl, C₁-C₄-Halogenalkyl, C₁-C₄-Alkoxy, C₁-C₄-Halogenalkoxy oder C₁-C₄-Alkoxy-carbonyl substituiertes Aryl oder Arylalkyl mit jeweils 6 oder 10 Kohlenstoffatomen im Arylteil und gegebenenfalls 1 bis 4 Kohlenstoffatomen im Alkylteil steht.

3. Verfahren nach Anspruch 1, dadurch gekennzeichnet, daß man die erste Umsetzungsstufe bei Temperaturen zwischen - 10°C und +40°C, insbesondere zwischen 0°C und 30°C, durchführt.

4. Verfahren nach Anspruch 1, dadurch gekennzeichnet, daß man die zweite Umsetzungsstufe bei Temperaturen zwischen 30°C und 90°C, insbesondere zwischen 40°C und 80°C, durchführt.

5. Verfahren nach Anspruch 1, dadurch gekennzeichnet, daß als Iminothiokohlensäurediester der Formel (II) Methyliminothiokohlensäure-O,S-dimethylester, Methyliminothiokohlensäure-O-n-propylester-S-methylester oder Iminothiokohlensäure-O-n-propylester-S-(2-methoxyethyl)-ester eingesetzt wird.

6. Verfahren nach Anspruch 1, dadurch gekennzeichnet, daß als Carbazinsäureester der Formel (III) Carbazinsäure-ethylester, Carbazinsäurephenylester oder deren Hydrochloride oder Dihydrogensulfate eingesetzt werden.

7. Verfahren nach Anspruch 1, dadurch gekennzeichnet, daß als Verdünnungsmittel (in beiden Reaktionsstufen) Alkohole, insbesondere Methanol, Ethanol, n- oder i-Propanol, deren Gemische mit Wasser sowie reines Wasser verwendet werden.

8. Verfahren nach Anspruch 1, dadurch gekennzeichnet, daß in der ersten Umsetzungsstufe als Reaktionshilfsmittel eine Protonensäure, insbesondere Pivalinsäure, Essigsäure oder (wäßrige) Salzsäure eingesetzt wird.

9. Verfahren nach Anspruch 1, dadurch gekennzeichnet, daß in der zweiten Umsetzungssufe als Base ein Alkalimetallalkoholat oder -hydroxid - jeweils gegebenenfalls im entsprechenden Alkohol bzw. in Wasser gelöst - eingesetzt wird.

## Claims

1. Process for the preparation of alkoxytriazolinones of the general formula (I), 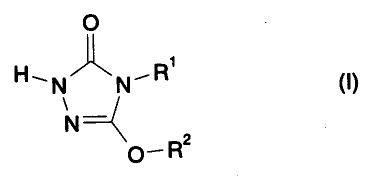 in which
R¹ represents in each case optionally substituted alkyl, alkenyl, alkinyl, cycloalkyl, cycloalkylalkyl, aryl or arylalkyl and
R² represents in each case optionally substituted alkyl, alkenyl, alkinyl, cycloalkyl, cycloalkylalkyl, aryl or arylalkyl,
characterized in that iminothiocarbonic diesters of the general formula (II) 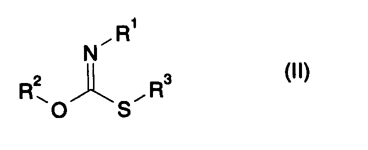 in which
R¹ and R² have the abovementioned meanings and
R³ represents in each case optionally substituted alkyl, aryl or arylalkyl,
are reacted with carbazinic esters of the general formula (III) 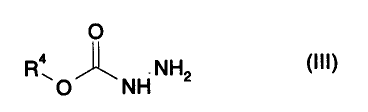 in which
R⁴ represents in each case optionally substituted alkyl, aryl or arylalkyl,
- or with acid addition products of compounds of the formula (III) -
if appropriate in the presence of a reaction auxiliary and if appropriate in the presence of a diluent at temperatures between -20°C and +50°C ("first reaction step") and the semicarbazide derivatives formed in this process of the general formula (IV) 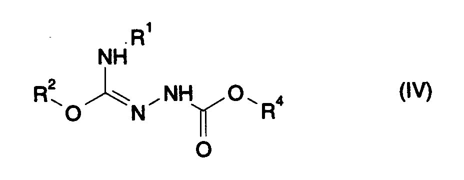 in which
R¹, R² and R⁴ have the abovementioned meanings,
- and/or the corresponding tautomeric compounds -
are subjected to a cyclizing condensation reaction, at temperatures between 20°C and 100°C, if appropriate after intermediate isolation, if appropriate in the presence of a base and if appropriate in the presence of a diluent ("second reaction step").

2. Process according to Claim 1, characterized in that compounds of the formula (I) are prepared in which
R¹ represents alkyl, alkenyl or alkinyl, each of which has up to 6 carbon atoms and each of which is optionally substituted by halogen or C₁-C₄-alkoxy, or represents cycloalkyl or cycloalkylalkyl, each of which has 3 to 6 carbon atoms in the cycloalkyl moiety and, if appropriate, 1 to 4 carbon atoms in the alkyl moiety and each of which is optionally substituted by halogen or C₁-C₄-alkyl, or represents aryl or arylalkyl, each of which has 6 or 10 carbon atoms in the aryl moiety and, if appropriate, 1 to 4 carbon atoms in the alkyl moiety and each of which is optionally substituted by carboxyl, cyano, nitro, halogen, C₁-C₄-alkyl, C₁-C₄-halogenoalkyl, C₁-C₄-alkoxy, C₁-C₄-halogenoalkoxy or C₁-C₄-alkoxy-carbonyl, and
R² represents alkyl, alkenyl or alkinyl, each of which has up to 6 carbon atoms and each of which is optionally substituted by halogen or C₁-C₄-alkoxy, or represents cycloalkyl or cycloalkylalkyl, each of which has 3 to 6 carbon atoms in the cycloalkyl moiety and, if appropriate, 1 to 4 carbon atoms in the alkyl moiety and each of which is optionally substituted by halogen or C₁-C₄-alkyl, or represents aryl or arylalkyl, each of which has 6 or 10 carbon atoms in the aryl moiety and, if appropriate, 1 to 4 carbon atoms in the alkyl moiety and each of which is optionally substituted by carboxyl, cyano, nitro, halogen, C₁-C₄-alkyl, C₁-C₄-halogenoalkyl, C₁-C₄-alkoxy, C₁-C₄-halogenoalkoxy or C₁-C₄-alkoxy-carbonyl.

3. Process according to Claim 1, characterized in that the first reaction step is carried out at temperatures between - 10°C and +40°C, in particular between 0°C and 30°C.

4. Process according to Claim 1, characterized in that the second reaction step is carried out at temperatures between 30°C and 90°C, in particular between 40°C and 80°C.

5. Process according to Claim 1, characterized in that the iminothiocarbonic diester of the formula (II) employed is O,S-dimethyl methyliminothiocarbonate, O-n-propyl S-methyl methyliminothiocarbonate or O-n-propyl S-(2-methoxyethyl) iminothiocarbonate.

6. Process according to Claim 1, characterized in that the carbazinic ester of the formula (III) employed is ethyl carbazinate, phenyl carbazinate or their hydrochlorides or dihydrogen sulphates.

7. Process according to Claim 1, characterized in that the diluents used (in both reaction steps) are alcohols, in particular methanol, ethanol or n- or i-propanol, mixtures of these with water, as well as pure water.

8. Process according to Claim 1, characterized in that the reaction auxiliary employed in the first reaction step is a protonic acid, in particular pivalic acid, acetic acid or (aqueous) hydrochloric acid.

9. Process according to Claim 1, characterized in that the base employed in the second reaction step is an alkali metal alcoholate or alkali metal hydroxide, in each case dissolved, if appropriate, in an appropriate alcohol or in water.

## Revendications

1. Procédé de production d'alkoxytriazolinones de formule générale (I) 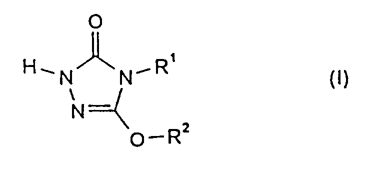 dans laquelle
R¹ représente un groupe alkyle, alcényle, alcynyle, cycloalkyle, cycloalkylalkyle, aryle ou arylalkyle dont chacun est éventuellement substitué et
R² représente un groupe alkyle, alcényle, alcynyle, cycloalkyle, cycloalkylalkyle, aryle ou arylalkyle dont chacun est éventuellement substitué,
caractérisé en ce qu'on fait réagir des diesters d'acides iminothiocarboniques de formule générale (II) 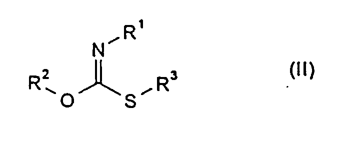 dans laquelle
R¹ et R² ont les définitions indiquées ci-dessus et
R³ est un groupe alkyle, aryle ou arylalkyle dont chacun est éventuellement substitué,
avec des esters d'acide carbazique de formule générale (III) 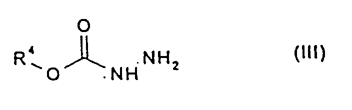 dans laquelle
R⁴ est un groupe alkyle, aryle ou arylalkyle dont chacun est éventuellement substitué,
- ou avec des produits d'addition d'acides de composés de formule (III) -
le cas échéant en présence d'un auxiliaire de réaction et en la présence éventuelle d'un diluant à des températures comprises entre -20°C et +50°C ("premier stade de réaction") et on condense par cyclisation ("second stade de réaction") les dérivés de semicarbazide ainsi formés de formule générale (IV) 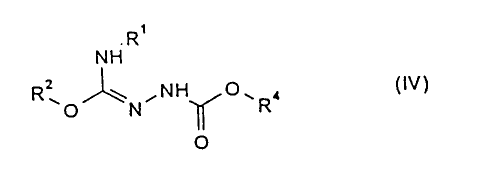 dans laquelle
R¹, R² et R⁴ ont les définitions indiquées ci-dessus,
- et/ou les composés tautomères qui y correspondent -,
le cas échéant après isolement intermédiaire, éventuellement en présence d'une base et en la présence éventuelle d'un diluant, à des températures comprises entre 20°C et 100°C.

2. Procédé suivant la revendication 1, caractérisé par la production de composés de formule (I) dans laquelle
R¹ représente un groupe alkyle, un groupe alcényle ou un groupe alcynyle ayant chacun jusqu'à 6 atomes de carbone et chacun étant substitué, le cas échéant, par un halogène ou un reste alkoxy en C₁ à C₄, un groupe cycloalkyle ou un groupe cycloalkylalkyle ayant chacun 3 à 6 atomes de carbone dans la partie cycloalkyle et, le cas échéant, 1 à 4 atomes de carbone dans la partie alkyle et dont chacun est éventuellement substitué par un halogène ou un reste alkyle en C₁ à C₄, ou bien un groupe aryle ou un groupe arylalkyle ayant chacun 6 ou 10 atomes de carbone dans la partie aryle et, le cas échéant, 1 à 4 atomes de carbone dans la partie alkyle et chacun étant substitué, le cas échéant, par un reste carboxy, cyano, nitro, halogéno, alkyle en C₁ à C₄, halogénalkyle en C₁ à C₄, alkoxy en C₁ à C₄, halogénalkoxy en C₁ à C₄ ou (alkoxy en C₁ à C₄)-carbonyle, et
R² représente un groupe alkyle, un groupe alcényle ou un groupe alcynyle ayant chacun jusqu'à 6 atomes de carbone et dont chacun est éventuellement substitué par un halogène ou un reste alkoxy en C₁ à C₄, un groupe cycloalkyle ou un groupe cycloalkylalkyle ayant chacun 3 à 6 atomes de carbone dans la partie cycloalkyle et, le cas échéant, 1 à 4 atomes de carbone dans la partie alkyle et dont chacun est éventuellement substitué par un halogène ou un reste alkyle en C₁ à C₄, ou bien un groupe aryle ou un groupe arylalkyle ayant chacun 6 ou 10 atomes de carbone dans la partie aryle et, le cas échéant, 1 à 4 atomes de carbone dans la partie alkyle et dont chacun est substitué, le cas échéant, par un reste carboxy, cyano, nitro, halogéno, alkyle en C₁ à C₄, halogénalkyle en C₁ à C₄, alkoxy en C₁ à C₄, halogénalkoxy en C₁ à C₄ ou (alkoxy en C₁ à C₄)-carbonyle.

3. Procédé suivant la revendication 1, caractérisé en ce qu'on conduit le premier stade de réaction à des températures comprises entre -10°C et +40°C, notamment entre 0°C et 30°C.

4. Procédé suivant la revendication 1, caractérisé en ce qu'on conduit le second stade de réaction à des températures comprises entre 30°C et 90°C, notamment entre 40°C et 80°C.

5. Procédé suivant la revendication 1, caractérisé en ce qu'on utilise comme diester d'acide iminothiocarbonique de formule (II) l'ester de O,S-diméthyle d'acide méthyliminothiocarbonique, l'ester de O-n-propyle et S-méthyle d'acide méthyliminothiocarbonique ou l'ester de O-n-propyle et S-(2-méthoxyéthyle) d'acide iminothiocarbonique.

6. Procédé suivant la revendication 1, caractérisé en ce qu'on utilise comme ester d'acide carbazique de formule (III) l'ester éthylique d'acide carbazique, l'ester phénylique d'acide carbazique ou leurs chlorhydrates et dihydrogénosulfates.

7. Procédé suivant la revendication 1, caractérisé en ce qu'on utilise comme diluants (dans les deux stades de réaction) des alcools, en particulier le méthanol, l'éthanol, le n-propanol ou l'isopropanol, leurs mélanges avec l'eau ainsi que l'eau pure.

8. Procédé suivant la revendication 1, caractérisé en ce qu'on utilise comme auxiliaire de réaction dans le premier stade de réaction un acide protonique, en particulier l'acide pivalique, l'acide acétique ou l'acide chlorhydrique (aqueux).

9. Procédé suivant la revendication 1, caractérisé en ce qu'on utilise comme base dans le deuxième stade de réaction un alcoolate ou hydroxyde de métal alcalin, chacun dissous, le cas échéant, dans l'alcool correspondant ou, respectivement, dans l'eau.
